# EUROPEAN PATENT APPLICATION

(11) **EP 3 338 575 A1**
(43) Date of publication of application: **27.06.2018**
(21) Application number: 17200929.2
(22) Date of filing: 09.11.2017
(51) Int. Cl.: A41D 13/12, A41D 13/11, A41D 13/002

(54) **MASK FOR SURGERY**

(30) Priority: 26.12.2016 KR 20160179370
(71) Applicant: Caleb Co., Ltd., Wonju-si, Gangwon-do 26311 (KR)
(72) Inventor: YOO, Kyungkil, 05236 Seoul (KR)
(74) Representative: RatnerPrestia

(57) **Abstract**

A surgical mask includes a hooded cover unit including a head cover and a shoulder cover, the head cover fully covering the head of a user and having an opening for opening a predetermined area around an eye, the nose, and the mouth of the face of the user, and the shoulder cover extended from the head cover, for covering the neck and a shoulder of the user, a fixing unit including a U-shaped support frame and an elastic band, the support frame disposed inside the hooded cover unit and extended curvedly along left and right sides of the head, and the elastic band connected to both ends of the support frame and extended along a front and rear of the head, a rotation frame engaged rotatably with both ends of the support frame, disposed outside the head cover, and extended curvedly along a front of the face, and a transparent cover shield mounted to the rotation frame, for covering an upper part of the face. When the rotation frame is at a first position, the transparent cover shield is disposed at a position corresponding to the face of the user and covers the opening of the head cover, and when the rotation frame is at a second position by upward rotation, the transparent cover shield exposes a part of the opening.

## Description

### BACKGROUND OF THE INVENTION

### Field of the Invention

The present disclosure relates to a surgical mask, and more particularly, to a surgical mask for protecting a user (or medical personnel) and a patient from viruses in a surgery environment by minimizing exposure of the body of the user, and making the user feel refreshed by introducing air in a part of the covered body.

### Discussion of the Related Art

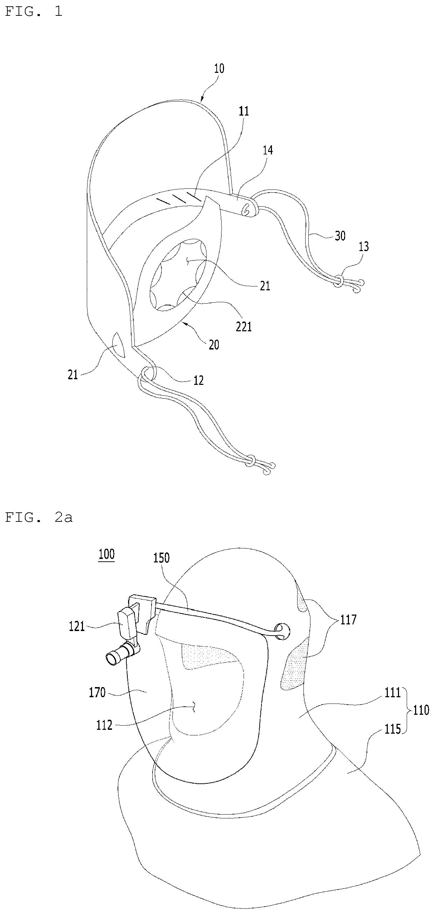

In general, a medical personnel wears an oral mask during a medical operation or procedure in order to prevent spatter of saliva from his or her mouth. Referring to FIG. 1, a conventional oral mask includes a chin support 20 for supporting the chin of a user, a cover 10 formed of a transparent film material, and a fixing unit 30 with which to both ends of the cover 10 on the ears of the user. Therefore, when the user wears the oral mask, the oral mask prevents the wearer from spattering his or her saliva to a patient during conversation or breathing, while stably supporting the chin of the user and thus relieving pressure or pain caused by wearing of the oral mask.

As described above, the primary purpose of the conventional oral mask lies in protecting a patient from viruses. However, as the fields of surgery have been increased and surgery technology has been developed, various virus infectants exist in a real surgery environment. Thus, there is an increasing need for protecting a user as well as a patient. For example, if body fluids or secretions from a patient contact the exposed skin of a medical personnel such as the face or neck, there is a risk of virus infection.

Moreover, the user should wear a mask in an operating room until surgery is completed. As an operation takes longer, the resulting difficult ventilation into the surgery mask causes the user to sweat. Thus, the user has no way but to endure the poor surgery environment.

Accordingly, there is a pressing need for a surgical mask for perfectly protecting a medical personnel and suppressing sweat generation during a long operation.

### SUMMARY OF THE INVENTION

Accordingly, to overcome limitations and disadvantages of the related art, an aspect of the present disclosure is to provide a surgical mask for perfectly protecting a user (or medical personnel) from various virus infectants in a surgery environment.

Another object of the present disclosure is to provide a surgical mask for suppressing sweat generation of a user during a surgical operation by forced ventilation into the surgical mask.

Additional advantages, objects, and features of the present disclosure will be set forth in part in the description which follows and in part will become apparent to those having ordinary skill in the art upon examination of the following or may be learned from practice of the present disclosure. The objectives and other advantages of the present disclosure may be realized and attained by the structure particularly pointed out in the written description and claims hereof as well as the appended drawings.

To achieve these objects and other advantages and in accordance with the purpose of the present disclosure, as embodied and broadly described herein, a surgical mask includes a hooded cover unit including a head cover and a shoulder cover, the head cover fully covering the head of a user and having an opening for opening a predetermined area around an eye, the nose, and the mouth of the face of the user, and the shoulder cover extended from the head cover, for covering the neck and a shoulder of the user, a fixing unit including a U-shaped support frame and an elastic band, the support frame disposed inside the hooded cover unit and extended curvedly along left and right sides of the head, and the elastic band connected to both ends of the support frame and extended along a front and rear of the head, a rotation frame engaged rotatably with both ends of the support frame, disposed outside the head cover, and extended curvedly along a front of the face, and a transparent cover shield mounted to the rotation frame, for covering an upper part of the face. When the rotation frame is at a first position, the transparent cover shield is disposed at a position corresponding to the face of the user and covers the opening of the head cover, and when the rotation frame is at a second position by upward rotation, the transparent cover shield exposes a part of the opening.

The shoulder cover may have one part on one side of a cut line, attached to the other part on the other side of the cut line.

The elastic band may be exposed outward through a hole formed on a rear of the head cover.

A cap member may be provided at each of both ends of the rotation frame, for being engaged with one end of the support frame, each of the support frame and the rotation frame may include a hollow hole in the form of a pipe, and when the cap members of the rotation frame are engaged with both ends of the support frame, the hollow hole of the rotation frame may communicate with the hollow hole of the support frame.

A cooling fan member may be mounted on a portion of the support frame, for introducing air into the hollow hole of the support frame, a plurality of through holes may be formed at every predetermined interval in each of the support frame and the rotation frame, and air may flow along the hollow holes of the support frame and the rotation frame by operation of the cooling fan member and may be discharged through the through holes of the support frame and the through holes of the rotation frame.

The surgical mask may further include a light unit including a light source and configured to adjust a light projection direction of the light source, and the light unit may be mounted on the rotation frame.

The rotation frame may be detachably engaged with both ends of the support frame, and when the rotation frame is detached from the support frame, the rotation frame and the transparent cover shield mounted to the rotation frame may be removed together from the surgical mask.

The surgical mask may further include a head light including a light unit and a head band worn around the head of the user, the light unit including a light source and configured to adjust a light projection direction of the light source. After the rotation frame and the transparent cover shield are removed from the surgical mask, the head light may be worn around the head.

It is to be understood that both the foregoing general description and the following detailed description of the present disclosure are exemplary and explanatory and are intended to provide further explanation of the present disclosure as claimed.

### BRIEF DESCRIPTION OF THE DRAWINGS

The accompanying drawings, which are included to provide a further understanding of the present disclosure and are incorporated in and constitute a part of this application, illustrate embodiment(s) of the present disclosure and together with the description serve to explain the principle of the present disclosure. In the drawings:
FIG. 1 is a perspective view illustrating a conventional oral mask according to an embodiment of the present disclosure;
FIG. 2A is a perspective view illustrating a surgical mask according to an embodiment of the present disclosure;
FIG. 2B is a front view illustrating the surgical mask illustrated in FIG. 2A;
FIG 2C is a side view illustrating the surgical mask illustrated in FIG. 2A;
FIG. 2D is a rear view illustrating the surgical mask illustrated in FIG. 2A;
FIG. 3 is a side view illustrating the surgical mask, when a rotation frame is placed at a second position by upward rotation;
FIG. 4 is a conceptual view illustrating a hooded cover unit according to another embodiment of the present disclosure;
FIG. 5A is a perspective view illustrating a U-shaped support frame in the surgical mask according to the present disclosure;
FIG. 5B is a front view illustrating the U-shaped support frame illustrated in FIG. 5A;
FIG. 6A is a perspective view illustrating a rotation frame in the surgical mask according to the present disclosure;
FIG. 6B is a plan view illustrating the rotation frame illustrated in FIG. 6A;
FIG. 7 is a perspective view illustrating the U-shaped support frame with a cooling fan member mounted thereto;
FIG. 8 is a conceptual view illustrating discharge of air generated by the cooling fan member through through holes formed in the support frame and the rotation frame along hollow holes of the support frame and the rotation frame; and
FIG. 9 is a perspective view illustrating a head light in a surgical mask according to another embodiment of the present disclosure.

### DETAILED DESCRIPTION OF THE INVENTION

Objects, advantages, and technical structures for achieving them will become apparent upon examination of the following detailed description of embodiments of the present disclosure as well as the attached drawings. In the description of the present disclosure, a detailed description of known functions or configurations will be omitted lest it should obscure the subject matter of the present disclosure. The terms as set forth herein are defined in consideration of the structures, roles, and functions of the present disclosure, and may vary according to the intent of a user and an operator, or customs.

However, the present disclosure is not limited to the disclosed embodiments. Rather, the present disclosure may be implemented in various other ways. The embodiments are provided to make the disclosure of the present disclosure comprehensive and help those skilled in the art to comprehensively understand the scope of the present disclosure, and the present disclosure is defined only by the appended claims. Therefore, the definition should be made based on the overall contents of the specification.

Throughout the specification, when it is said that some part "includes" or "has" a component, this means that the part may further include another component, not excluding the other component, unless otherwise specified.

With reference to the attached drawings, preferred embodiments of the present disclosure will be described in great detail.

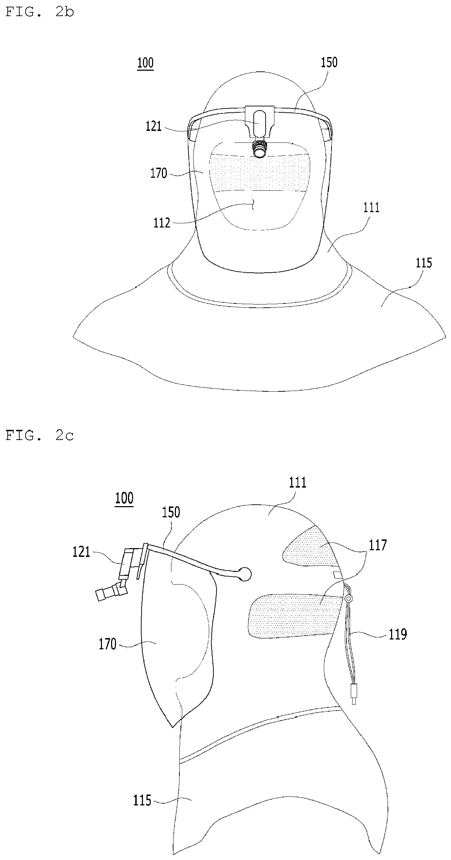

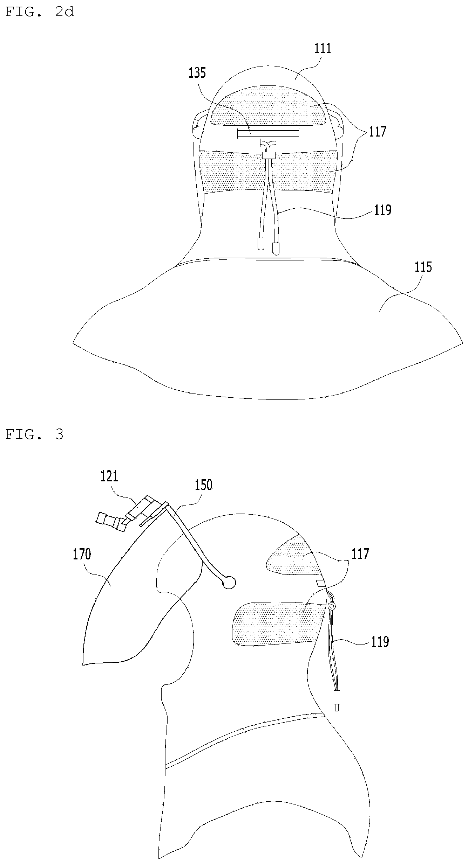

FIG. 2A is a perspective view illustrating a surgical mask according to an embodiment of the present disclosure, FIG. 2B is a front view illustrating the surgical mask illustrated in FIG. 2A, FIG 2C is a side view illustrating the surgical mask illustrated in FIG. 2A, and FIG. 2D is a rear view illustrating the surgical mask illustrated in FIG. 2A. Referring to FIGS. 2A to 2D, a surgical mask 100 according to an embodiment of the present disclosure is configured to include a hooded cover unit 110, a fixing unit 130, a rotation frame 150, and a transparent cover shield 170.

The hooded cover unit 110 includes a head cover 111 having an opening 112 that opens a predetermined area around the eyes, nose, and mouth on a user's face, for fully covering the head of the user and, and a shoulder cover 115 extended from the head cover 111, for covering the neck and shoulders of the user. The head cover 111 may be integrated with the shoulder cover 115. As illustrated, the hooded cover unit 1110 may be formed of an elastic material to closely contact the head, neck, and shoulders of the user. As far as it is a generally used material capable of preventing harmful materials included in ambient air from contacting the skin of a user, any material is available for the hooded cover unit 110.

The head cover 111 is a part into which the head of the user is inserted. The circular opening 112 is formed on the front surface of the head cover 111. The eyes, nose, and mouth can be exposed through the circular opening 112. The circular opening 112 may vary in size. Further, as illustrated in FIGS. 2A to 2D, ventilation sheets 117 may be provided on the rear surface of the head cover 111 so that convection of ambient air into the head cover 111 may occur. Further, as illustrated in FIGS. 2C and 2D, the head cover 111 may further include a length adjustment strap 119.

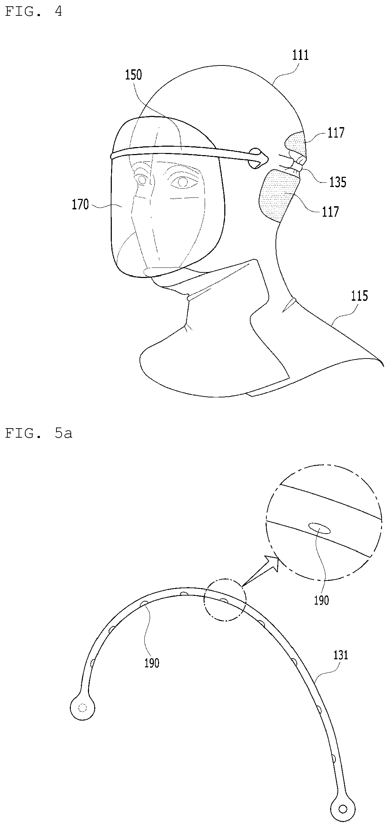

Meanwhile, the shoulder cover 115 is a part placed on the shoulders of the user, covering the user's neck. The shoulder cover 115 is preferably integrated with the head cover 111. For the convenience of wearing, the shoulder cover 115 may be partially cut along a line, and one part of the shoulder cover 115 on one side of the line may be attached to the other part of the shoulder cover 115 on the other side of the line, as illustrated in FIG. 4. For example, the one cut part may be attached onto the other cut part by a general fixing means such as a snap fastener or a Velcro closure.

Meanwhile, the hooded cover unit 110 further includes the fixing unit 130 inside it, which supports the shape of the hooded cover unit 110 and, when the hooded cover unit 110 is worn, is mounted on the user's head. That is, a head-shaped frame maintained by the fixing unit 130 keeps the shape of the hooded cover unit 110. Specifically, the fixing unit 130 includes a U-shaped support frame 131 extended curvedly along the left and right sides of the user's head, inside the hooded cover unit 110, and an elastic band 135 extended along the front and rear sides of the head and connected to both ends of the support frame 131.

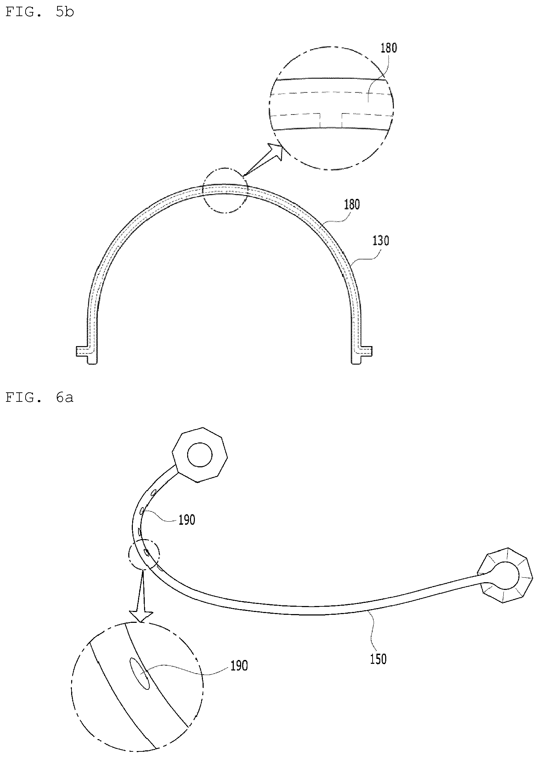

FIG. 5A is a perspective view illustrating the U-shaped support frame 131 in the surgical mask 100 of the present disclosure, and FIG. FIG. 5B is a front view illustrating the U-shaped support frame illustrated in FIG. 5A. Referring to FIGS. 5A and 5B, the U-shaped support frame 131 is worn around the head of the user so that one end of the U-shaped support frame 131 may be disposed in the vicinity of, for example, the right temple of the user and the other end of the U-shaped support frame 131 may be disposed in the vicinity of, for example, the left temple of the user. The support frame 131 is preferably formed of an elastic material so that the distance between both ends of the support frame 131 may be flexibly controlled according to a user. The elastic band 135 is provided at both ends of the support frame 131 in order to fix the surgical mask 100 of the present disclosure to the head. As illustrated in FIG. 2D, the elastic band 135 may be configured to be partially exposed outward from the head cover 111 through a hole formed on the rear of the head cover 111.

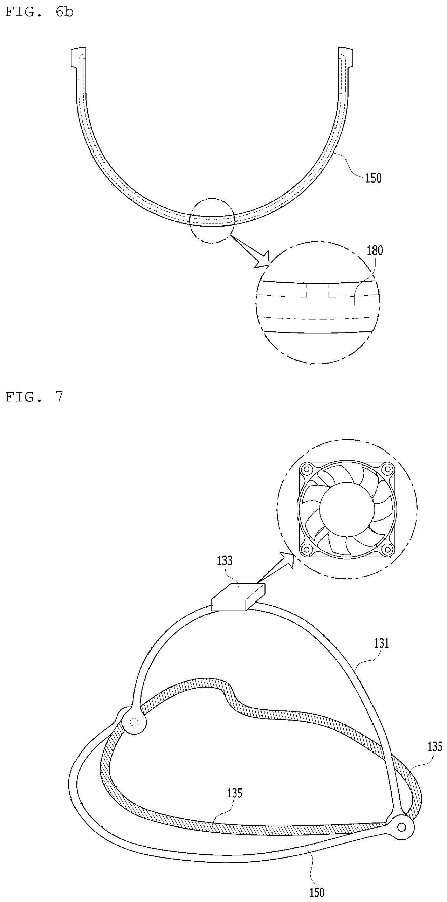

Meanwhile, the surgical mask 100 according to the embodiment of the present disclosure includes the rotation frame 150 extended along the front of the face and rotatably engaged with both ends of the support frame 131, outside the head cover 111. FIG. 6A is a perspective view illustrating the rotation frame 150 in the surgical mask 100 of the present disclosure, and FIG. 6B is a plan view illustrating the rotation frame 150 illustrated in FIG. 6A. Referring to FIGS. 6A and 6B, cap members 151 are provided at both ends of the rotation frame 150, to be engaged with both ends of the support frame 131. Each cap member 151 accommodates a male member protruding from one end of the support frame 131.

Meanwhile, the transparent cover shield 170 is mounted to the rotation frame 150, for covering an upper part of the face. As illustrated in FIG. 2A, the transparent cover shield 170 may be formed in a size fully covering the opening 112 and a shape corresponding to the contour of the face. Further, an area corresponding to the chin of the user at the bottom end of the transparent cover shield 170 may be curved along the contour of the chin.

A light unit 121 may be mounted on the rotation frame 150. The light unit 121 may be configured to include a light source and adjust a light projection direction of the light source. Referring to FIG. 2A, the light unit 121 may be mounted, for example, on a front center of the rotation frame 150. The light unit 121, which is a tool that illuminates light onto a body part for surgery, is engaged with the rotation frame 150 in such a manner that the light unit 121 may be rotatable at 360 degrees. Accordingly, the user may adjust the light projection direction by manually manipulating the light unit 121 during the course of surgery.

According to an embodiment, the rotation frame 150 is configured to be rotatable upward and downward with respect to both ends of the rotation frame 150 engaged with the support frame 131. Herein, the rotation frame 150 is rotated between a first position and a second position. The first position is a position at which the rotation frame 150 may cover the face when the user wears the surgical mask 100 of the present disclosure and performs surgery, for example, as illustrated in FIG. 2C. When the rotation frame 150 is at the first position, the transparent cover shield 170 is disposed at a position corresponding to the face of the user and covers the opening 112 of the head cover 111. On the other hand, the second position is a position at which the rotation frame 150 is disposed to expose the mouth or nose when needed, in the state where the user wears the surgical mask 100 of the present disclosure, for example, as illustrated in FIG. 3. When the rotation frame 150 is disposed at the second position, the transparent cover shield 170 partially exposes the opening 112 of the head cover 111. The rotation frame 150 may be rotated by the user's manual manipulation of a rotation lever (not shown) formed on a cap member 151 of the rotation frame 150.

Now, a description will be given of structural features of the support frame 131 and the rotation frame 150 according to an embodiment of the present disclosure.

As illustrated in FIG. 5A and 5B, one of the structural features of the support frame 131 in the fixing unit 130 is that a hollow hole 180 is formed in the shape of a pipe inside the support frame 131 and communicates with the outside at both ends of the support frame 131. Another of the structural features of the support frame 131 is that a plurality of through holes 190 are formed at every predetermined interval on an inner surface of the support frame 131, that is, a surface of the support frame 131 facing the scalp of the user and communicate with the hollow hole 180 in the support frame 131.

Meanwhile, one of the structural features of the rotation frame 150 is that a hollow hole 180 is formed in the shape of a pipe inside the rotation frame 150 and communicates with the outside at both ends of the rotation frame 150. Another of the structural features of the support frame 131 is that a plurality of through holes 190 are formed at every predetermined interval on an inner surface of the rotation frame 150, that is, a surface of the rotation frame 150 facing the face of the user and communicate with the hollow hole 180 in the rotation frame 150.

As described above, the rotation frame 150 is rotatably engaged with both ends of the support frame 131. When the cap members 151 of the rotation frame 150 are engaged with both ends of the support frame 131, the hollow hole 180 of the rotation frame 150 communicates with the hollow hole 180 of the support frame 131.

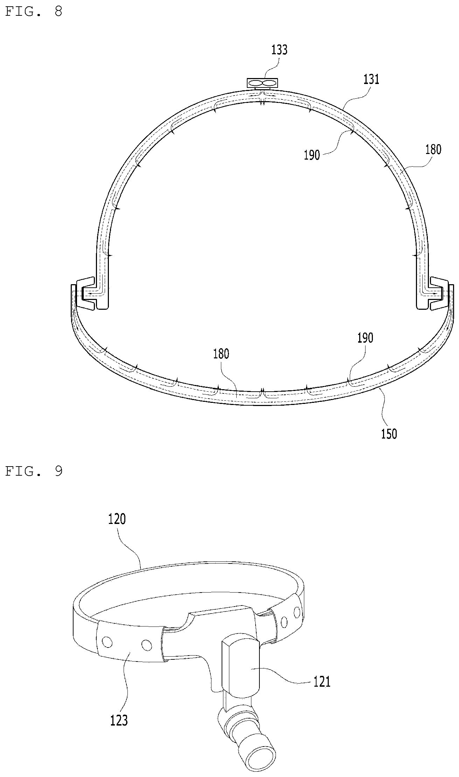

Meanwhile, a cooling fan member 133 is mounted onto one portion of the support frame 131 to inject air into the hollow hole 180 of the support frame 131. FIG. 7 is a perspective view illustrating the cooling fan member 133 mounted on the U-shaped support frame 131. The cooling fan member 133 is a part having a fan installed in a casing, in which the fan receives power from a power supply such as a battery and thus rotates, thereby producing an air flow. The cooling fan member 133 is preferably installed at, but not limited to, a center of the support frame 131 so that air may flow uniformly to both ends of the support frame 131. When the cooling fan member 133 is mounted on the support frame 131, an air outlet of the cooling fan member 133 communicates with the hollow hole 180 of the support frame 131. FIG. 8 is a conceptual view illustrating discharge of air generated by the cooling fan member 133 through the through holes 190 formed in each frame along the hollow holes 180 of the support frame 131 and the rotation frame 150. Referring to FIG. 8, once air is primarily introduced into the hollow hole 180 of the support frame 131 by operation of the cooling fan member 133, the air flows along the hollow hole 180 of the support frame 131 and moves to both ends of the support frame 131. Herein, each time the air passes the through holes 190 formed in the support frame 131, the air is partially discharged outside the support frame 131 through the through holes 190, thus providing fresh air to the head of the user. Then, the air reaching both ends of the support frame 131 enters the hollow hole 180 of the rotation frame 150 communicating with the support frame 131. That is, the air reaching one end of the support frame 131 enters the hollow hole 180 of the rotation frame 150 through the cap member 151 at one end of the rotation frame 150, and the air reaching the other end of the support frame 131 enters the hollow hole 180 of the rotation frame 150 through the cap member 151 at the other end of the rotation frame 150. The introduced air flows through the hollow hole 180 of the rotation frame 150. Herein, each time the air passes the through holes 190 formed in the rotation frame 150, the air is partially discharged outside the rotation frame 150 through the through holes 190, thus providing fresh air to the face of the user.

The above-described configuration discharges air generated by the cooling fan member 133 to the body of the user through the frames 131 and 150 of the hooded cover unit 110, thereby suppressing sweat generation of the user during surgery.

In a surgery mask according to another embodiment of the present disclosure, the rotation frame 150 is detachably engaged with both ends of the support frame 131. The rotation frame 150 may be formed of an elastic material that enables the distance between both ends of the rotation frame 150 to be flexibly increased or decreased. Accordingly, the rotation frame 150 may be removed from the support frame 131 by widening the gap between both ends of the rotation frame 150 by external force and thus removing both ends of the rotation frame 150 from both ends of the support frame 131. When the rotation frame 150 is removed from the support frame 131, the transparent cover shield 170 mounted to the rotation frame 150 is also removed along with the rotation frame 150 from the surgical mask. Since the transparent cover shield 170 is generally not needed except for a situation in which blood spatters during surgery, the transparent cover shield 170 is configured to be selectively removed.

Meanwhile, a surgical mask according to another embodiment of the present disclosure may further include the head light 120 worn around the head of the user. FIG. 9 is a perspective view illustrating a head light in a surgical mask according to another embodiment of the present disclosure. Referring to FIG. 9, the head light 120 includes the light unit 121 having a light source and configured to adjust a light projection direction of the light source, and a flexible head band 123 worn around the head of the user. With the rotation frame 150 and the transparent cover shield 170 removed from the surgical mask, the user may wear the head light 120 around the head.

As is apparent from the foregoing description, a surgical mask according to an embodiment of the present disclosure provides a hooded cover unit configured to minimize exposure of the body of a user (or medical personnel), thereby perfectly protecting the user from various virus infectants in a surgery environment.

Further, the surgical mask is provided with a cooling fan member, and thus discharges air generated by a motor to the body of the user through a support frame extended cross the head of the user and a rotation frame extended cross the face of the user. As a consequence, sweat generation of the user is suppressed during surgery.

It will be apparent to those skilled in the art that various modifications and variations can be made in the present disclosure without departing from the spirit or scope of the present disclosure. Thus, it is intended that the present disclosure covers the modifications and variations of this present disclosure provided they come within the scope of the appended claims and their equivalents.

## Claims

1. A surgical mask, comprising:
a hooded cover unit including a head cover and a shoulder cover, the head cover fully covering the head of a user and having an opening for opening a predetermined area around an eye, the nose, and the mouth of the face of the user, and the shoulder cover extended from the head cover, for covering the neck and a shoulder of the user;
a fixing unit including a U-shaped support frame and an elastic band, the support frame disposed inside the hooded cover unit and extended curvedly along left and right sides of the head, and the elastic band connected to both ends of the support frame and extended along a front and rear of the head;
a rotation frame engaged rotatably with both ends of the support frame, disposed outside the head cover, and extended curvedly along a front of the face; and
a transparent cover shield mounted to the rotation frame, for covering an upper part of the face,
wherein when the rotation frame is at a first position, the transparent cover shield is disposed at a position corresponding to the face of the user and covers the opening of the head cover, and when the rotation frame is at a second position by upward rotation, the transparent cover shield exposes a part of the opening.

2. The surgical mask according to claim 1, wherein the shoulder cover has one part on one side of a cut line, attached to the other part on the other side of the cut line.

3. The surgical mask according to claim 1, wherein the elastic band is exposed outward through a hole formed on a rear of the head cover.

4. The surgical mask according to claim 1, wherein a cap member is provided at each of both ends of the rotation frame, for being engaged with one end of the support frame, each of the support frame and the rotation frame includes a hollow hole in the form of a pipe, and when the cap members of the rotation frame are engaged with both ends of the support frame, the hollow hole of the rotation frame communicates with the hollow hole of the support frame.

5. The surgical mask according to claim 4, wherein a cooling fan member is mounted on a portion of the support frame, for introducing air into the hollow hole of the support frame, a plurality of through holes are formed at every predetermined interval in each of the support frame and the rotation frame, and air flows along the hollow holes of the support frame and the rotation frame by operation of the cooling fan member and is discharged through the through holes of the support frame and the through holes of the rotation frame.

6. The surgical mask according to claim 1, further comprising a light unit including a light source and configured to adjust a light projection direction of the light source,
wherein the light unit is mounted on the rotation frame.

7. The surgical mask according to claim 1, wherein the rotation frame is detachably engaged with both ends of the support frame, and when the rotation frame is detached from the support frame, the rotation frame and the transparent cover shield mounted to the rotation frame are removed together from the surgical mask.

8. The surgical mask according to claim 7, further comprising a head light including a light unit and a head band worn around the head of the user, the light unit including a light source and configured to adjust a light projection direction of the light source,
wherein after the rotation frame and the transparent cover shield are removed from the surgical mask, the head light is worn around the head.
